# EUROPÄISCHE PATENTANMELDUNG

(11) **EP 2 687 190 A1**
(43) Veröffentlichungstag der Anmeldung: **22.01.2014**
(21) Anmeldenummer: 13002836.8
(22) Anmeldetag: 01.06.2013
(51) Int. Cl.: A61F 2/36, B23K 35/00, A61F 2/30

(54) **Orthopädisches Implantat**

(30) Priorität: 20.07.2012 DE 102012014345
(71) Anmelder: ZM Präzisionsdentaltechnik GmbH, 18055 Rostock (DE)
(72) Erfinder: Mitrovic, Milija, D-18055 Rostock (DE)
(74) Vertreter: Hansmann, Dierk

(57) **Zusammenfassung**

Ein orthopädisches Implantat in Form einer Hüftgelenk-Endoprothese besteht aus einem Kopf (3), der auf einen Verankerungsschaft (1) aufgesetzt ist, der seinerseits in einen Knochen einführbar und in diesem verankerbar ist. Der Kopf weist eine innere sacklochartige Ausnehmung (6) auf und der Verankerungsschaft ist mit einem Zapfen (2) zum Einstecken in diese Ausnehmung versehen. In die Ausnehmung des Kopfes ist dabei eine Hülse (5) eingelötet, über die der Kopf auf den Zapfen aufsetzbar ist. Der Kopf besteht aus einer Keramik auf der Basis von Zirkoniumdioxid, Aluminiumoxid oder Mischkeramik, während die Hülse aus einem metallischen Werkstoff besteht. Die Verbindung zwischen dem Kopf und der Hülse wird durch ein in einem keramischen Brand verfestigtes silikatisches Keramiklot hergestellt.

## Beschreibung

Die Erfindung betrifft ein orthopädisches Implantat in Form einer femoralen Komponente einer Hüftgelenk-Endoprothese mit einem Kopf, der auf einen Verankerungsschaft aufgesetzt ist, der seinerseits in einen Knochen einführbar und in diesem verankerbar ist, wobei der Kopf eine innere sacklochartige Ausnehmung aufweist und der Verankerungsschaft mit einem Zapfen zum Einstecken in diese Ausnehmung versehen ist und wobei in die Ausnehmung des Kopfes eine metallische Hülse eingesetzt ist, über die der Kopf mit dem Zapfen verbindbar ist.

Die femorale Komponente einer Hüftgelenk-Endoprothese besteht im wesentlichen aus einem Schaft zur Verankerung im Kern des Röhrenknochens des Oberschenkels, wobei auf der proximalen Auflage des Schaftes ein angenähert kugelkalottenartiger Kopf zum Ersatz des erkrankten Hüftkopfes so aufgebracht ist, dass er mit dem Verankerungsschaft eine tragfähige Einheit bildet. Während bei manchen Endoprothesen dieser Art der Schaft mit dem Kopf aus artgleichem Material untrennbar miteinander verschweißt ist, besteht eine andere Art der Verbindung zwischen dem Prothesenkopf und dem Verankerungsschaft aus einer lösbaren Steckverbindung. Bei dieser ist ein tragender Zapfen in Form eines Konus am proximalen Ende des Prothesenverankerungsschaftes angeordnet, auf den wiederum der Prothesenkopf aufgesteckt ist. Eine solche Anordnung ist aus der DE 2 921 529 A1 bekannt geworden. Die Verbindung zwischen dem Kopf und dem tragenden Zapfen kann, wie bei dieser bekannten Anordnung vorgesehen, lösbar ausgebildet sein, wodurch es möglich ist, während der Operation je nach Indikation auf den tragenden Zapfen entweder nur einen Kopf mit größerem Durchmesser zum Ersatz des erkrankten Hüftkopfes aufzusetzen und dadurch eine sogenannte Hemiprothese zu implantieren, oder aber durch einen Kopf kleineren Durchmessers diesen mittels einer Ersatzpfanne zu einer Total-Hüftendoprothese zu vervollständigen.

Solche Hüftgelenk-Endoprothesen sind nur dann zuverlässig einsetzbar, wenn neben einer einwandfreien Verankerung des Prothesenschaftes im Femur auch die Langzeitfunktion des den Kopf tragenden Zapfenlagers und des Kopfes selbst gewährleistet ist. So besteht insbesondere bei den für Prothesenköpfe aus Oxidkeramik üblicherweise verwendeten konischen Steckverbindungen zur Herstellung einer mechanisch festen und schlupffreien Verklemmung zwischen dem konusförmigen Lagerzapfen des Verankerungsschaftes und dem Metall- oder Keramikkopf das Problem des Verlustes einer mechanisch sicheren Verbindung sowie der Zerstörung der Oberfläche der Werkstoffpaarung aus Kopf und Schaft, wodurch Korrosionsvorgänge ausgelöst werden können, die die Langzeitfunktion der gesamten Prothese infrage stellen können. Zudem kann eine unzureichende Passung des Keramikkopfes mit dem metallischen Zapfen zu erhöhten Spannungen in der Keramik führen, mit möglichem nachfolgenden Bruch der Implantatkomponente. Des Weiteren ist das Aufstecken eines Keramikkopfes im Rahmen der Wechseloperation auf einen in-situ belassenen Schaft kritisch zu sehen.

Aus diesem Grund stellt es eine bereits bekannte Maßnahme dar, im Keramikkopf einer Hüftgelenk-Endoprothese eine Metallhülse für das Fügen des Kopfes mit dem Zapfen vorzusehen. Dabei wird bei den bislang bekannten Anordnungen der eingangs genannten Art die Hülse entweder intraoperativ vom Operateur mit dem Kopf gefügt, oder aber sie wird präoperativ in diesen eingepreßt.

Aufgabe der Erfindung ist es, ein Implantat der eingangs genannten Art derart auszubilden, dass der Kopf und der Schaft derart präoperativ miteinander verbunden sind, dass eine feste, nicht lösbare Verbindung zwischen der Hülse und dem Kopf entsteht und dadurch unter anderem kein Abrieb der artikulierenden Materialien von Kopf und Schaft entstehen kann und kritische, durch den Fügevorgang verursachte Spannungen im Kopf vermieden werden.

Die Erfindung löst diese Aufgabe, indem sie vorsieht, dass bei einem derartigen Implantat die Hülse in den Kopf eingelötet ist.

Durch die integrierte Metallhülse werden kritische Spannungen im Kopf, die beim intraoperativen Fügevorgang des Kopfes auf den in der Regel metallischen konusförmigen Schaft auftreten können und die zu Rissen im Kopf führen können, weitestgehend vermieden. Dies ist insbesondere dann von signifikanter Bedeutung, wenn der Kopf aus einer Keramik, beispielsweise aus Aluminium- oder Zirkoniumdioxid, besteht.

Nachfolgend soll die Erfindung anhand eines in der Zeichnung dargestellten Ausführungsbeispiels näher erläutert werden. Es zeigen:
- Fig. 1: eine Hüftgelenk-Endoprothese in perspektivischer Darstellung,
- Fig. 2: eine Explosionsdarstellung der Anordnung gemäß Fig. 1

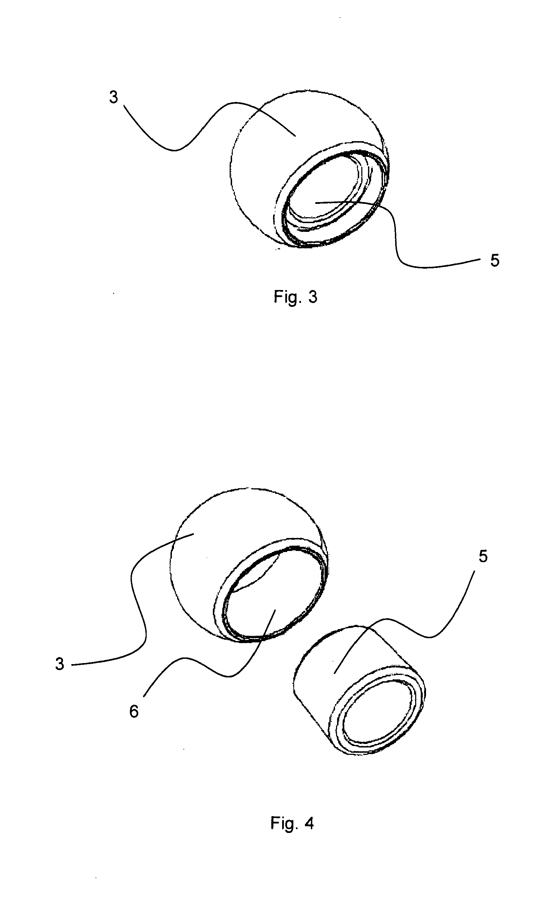

- Fig. 3: eine vergrößerte Darstellung des Kopfes der Hüftgelenk-Endoprothese gemäß Fig. 1 und
- Fig. 4: eine Explosionsdarstellung der Anordnung gemäß Fig. 3.

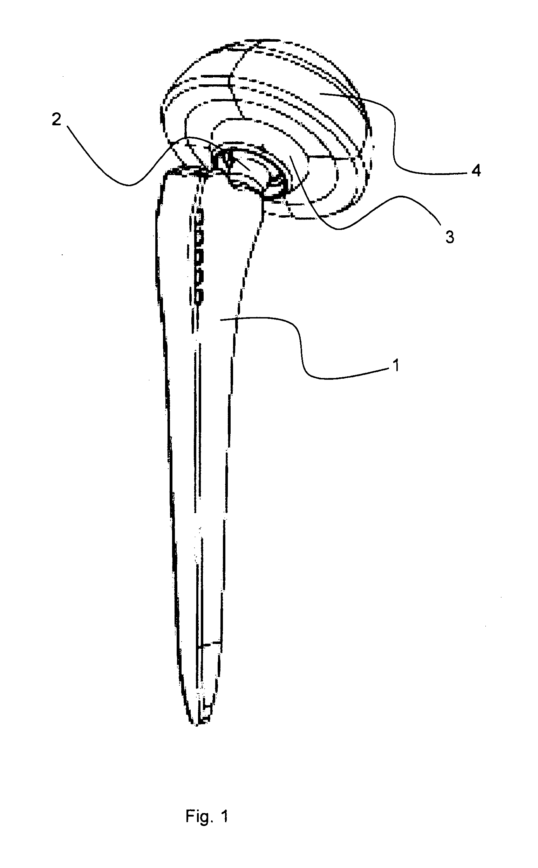

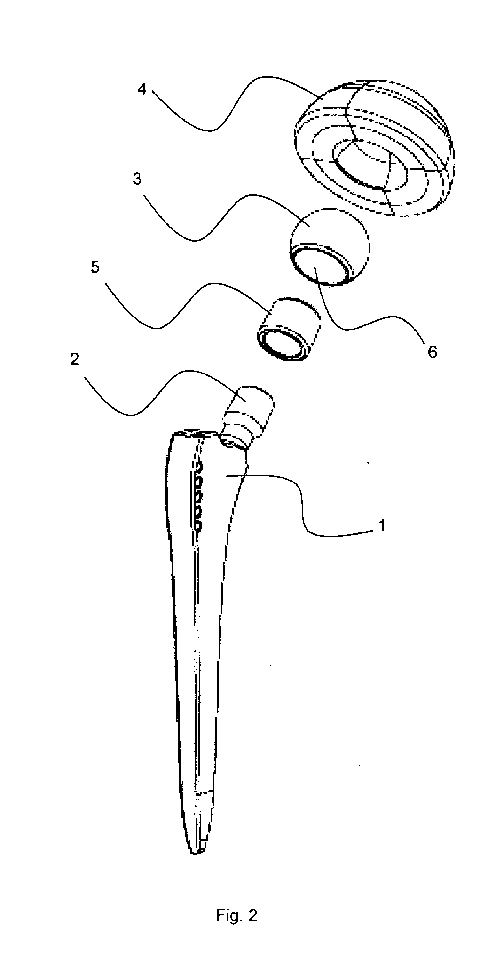

Die Darstellung gemäß der Figuren 1 und 2 zeigt eine femorale Komponente einer Hüftgelenk-Endoprothese mit einem Schaft 1 zur Verankerung im Kern des Röhrenknochen des Oberschenkels eines Patienten sowie mit einem auf eine proximale Auflage 2 des Schaftes 1 aufsetzbaren, angenähert kugelkalottenartig ausgebildeten Kopf 3 zum Ersatz des erkrankten Hüftkopfes des Patienten. Dieser Kopf 3 ist in eine Ersatzpfanne 4 einer Total-Hüftendoprothese eingesetzt.

Der Kopf 3 besteht im Fall des hier dargestellten Ausführungsbeispiels aus einer Oxidkeramik, und ist mit einer eingelöteten Hülse 4, die im Fall des hier dargestellten Ausführungsbeispiels aus einem Titanwerkstoff besteht, versehen. Zu diesem Zweck wird auf die Außenseite der Metallhülse 5 und/oder auf die Innenfläche einer sacklochartigen Bohrung 6 im Keramikkopf 3 ein Lot aufgebracht und es wird auf diese Weise nach dem Fügen eine feste, nicht lösbare Verbindung zwischen dem Keramikkopf 3 und der Metallhülse 5, die außer aus einem Titanwerkstoff zum Beispiel auch aus einer Kobalt-Chrom-Legierung (CoCr) bestehen kann, geschaffen. Das Löten erfolgt bereits beim Hersteller der Prothese und damit bereits weit vor der Operation aufgebracht. Bei dem hier beschriebenen Ausführungsbeispiel wird der Keramikkopf 3 durch ein Glaslot mit der Metallhülse 5 verbunden, wobei diese Verbindung durch ein in einem keramischen Brand verfestigtes silikatisches Keramiklot hergestellt wird. Während die Außenseite des Keramikkopfes 3 bei diesem Arbeitsgang nicht modifiziert wird, sind die mit dem Lot behandelten Areale der inneren Oberfläche des Keramikkopfes 3 komplett von der Metallhülse 5 überdeckt, so dass keine freie, mit der Umgebung interagierende modifizierte Keramikoberfläche besteht.

Der auf diese Weise fest mit der Metallhülse 5 verbundene Keramikkopf 3 wird anschließend intraoperativ direkt auf den metallischen Zapfen 2 aufgesteckt. Durch die eingelötete Metallhülse 5 werden kritische Spannungen im Keramikkopf 3, die ansonsten beim Fügevorgang des Kopfes 3 auf den metallischen Zapfen 2 auftreten können, zuverlässig vermieden. Der Kopf 3 artikuliert mit der künstlichen Pfanne 4 bzw. mit einem Pfanneneinsatz; ein Kontakt des Kopfes 3 zum umgebenden Knochen besteht nicht.

## Patentansprüche

1. Orthopädisches Implantat in Form einer femoralen Komponente einer Hüftgelenk-Endoprothese mit einem Kopf, der auf einen Verankerungsschaft aufgesetzt ist, der seinerseits in einen Knochen einführbar und in diesem verankerbar ist, wobei der Kopf eine innere sacklochartige Ausnehmung aufweist und der Verankerungsschaft mit einem Zapfen zum Einstecken in diese Ausnehmung versehen ist und wobei in die Ausnehmung des Kopfes eine metallische Hülse eingesetzt ist, über die der Kopf mit dem Zapfen verbindbar ist, **dadurch gekennzeichnet, dass** die Hülse (5) in den Kopf (3) eingelötet ist.

2. Implantat nach Anspruch 1, **dadurch gekennzeichnet, dass** der Kopf (3) aus einer Keramik auf der Basis von Zirkoniumdioxid besteht.

3. Implantat nach Anspruch 1, **dadurch gekennzeichnet, dass** der Kopf (3) aus einer Keramik auf der Basis von Aluminiumoxid besteht.

4. Implantat nach Anspruch 1, **dadurch gekennzeichnet, dass** der Kopf (3) aus einer Keramik auf der Basis von Mischkeramik besteht.

5. Implantat nach einem der Ansprüche 1 bis 4, **dadurch gekennzeichnet, dass** die Hülse (5) aus Reintitan besteht.

6. Implantat nach einem der Ansprüche 1 bis 4, **dadurch gekennzeichnet, dass** die Hülse (5) aus einer Titanlegierung besteht.

7. Implantat nach einem der Ansprüche 1 bis 4, **dadurch gekennzeichnet, dass** die Hülse (5) aus einer Kobalt-Chrom-Legierung besteht.

8. Implantat nach einem der Ansprüche 1 bis 7, **dadurch gekennzeichnet, dass** der Grundkörper (2) der femoralen Komponente (1) aus einer Titanlegierung besteht.

9. Implantat nach einem der Ansprüche 1 bis 7, **dadurch gekennzeichnet, dass** der Grundkörper (2) der femoralen Komponente (1) aus einer Kobalt-Chrom-Legierung besteht.

10. Implantat nach einem der Ansprüche 1 bis 7, **dadurch gekennzeichnet, dass** der Grundkörper (2) der femoralen Komponente (1) aus Edelstahl besteht.

11. Implantat nach einem der Ansprüche 1 bis 10, **dadurch gekennzeichnet, dass** der Kopf (3) durch ein Glaslot mit der Hülse (5) verbunden ist.

12. Implantat nach Anspruch 11, **dadurch gekennzeichnet, dass** die Verbindung durch ein in einem keramischen Brand verfestigtes silikatisches Keramiklot hergestellt ist.
